# EUROPEAN PATENT APPLICATION

(11) **EP 3 075 270 A1**
(43) Date of publication of application: **05.10.2016**
(21) Application number: 15161682.8
(22) Date of filing: 30.03.2015
(51) Int. Cl.: A24F 47/00

(54) **ATOMIZER AND ATOMIZER/LIQUID RESERVOIR PORTION FOR ELECTRONIC SMOKING DEVICE AND ELECTRONIC SMOKING DEVICE**

(71) Applicant: Fontem Holdings 1 B.V., 1083 HN Amsterdam (NL)
(72) Inventor: Pienemann, Thomas, 22761 Hamburg (DE)
(74) Representative: Gulde & Partner

(57) **Abstract**

The invention relates to an atomizer (140) and an atomizer/liquid reservoir portion for electronic smoking devices (110), and to an electronic smoking device (110). The atomizer/liquid reservoir portion and the electronic smoking device (110) each comprise the atomizer (140). In order to be able to efficiently atomize or vaporize liquids to be inhaled by a user of the electronic smoking device (110), the atomizing element of the atomizer (140) is an infrared light source (42) for vaporizing the liquid.

## Description

### FIELD OF INVENTION

The present invention relates generally to electronic smoking devices and in particular electronic cigarettes, as well as to atomizers and to atomizer/liquid reservoir portions for electronic smoking devices.

### BACKGROUND OF THE INVENTION

An electronic smoking device, such as an electronic cigarette (e-cigarette), typically has a housing accommodating an electric power source (e.g. a single use or rechargeable battery, electrical plug, or other power source), and an electrically operable atomizer. The atomizer vaporizes or atomizes liquid supplied from a reservoir and provides vaporized or atomized liquid as an aerosol. Control electronics control the activation of the atomizer. In some electronic cigarettes, an airflow sensor is provided within the electronic smoking device which detects a user puffing on the device (e.g., by sensing an under-pressure or an air flow pattern through the device). The airflow sensor indicates or signals the puff to the control electronics to power up the device and generate vapor. In other e-cigarettes, a switch is used to power up the e-cigarette to generate a puff of vapor.

Electrically operable atomizers, however, have a low efficiency when vaporizing liquid. Yet, the electrical energy available and e.g. storable in the electronic smoking device is limited, such that the available electrical energy may be depleted faster than desirable. Hence, it may be desirable to provide an electronic smoking device with an improved period of use.

### SUMMARY OF THE INVENTION

In accordance with one aspect of the present invention, there is provided an atomizer for an electronic smoking device, with an atomizing element that is adapted to atomize, e.g. by vaporization, liquid to be inhaled by a user of the electronic smoking device. Furthermore, in accordance with another aspect of the present invention, there is provided an atomizer/liquid reservoir portion for an electronic smoking device, with a liquid reservoir and an atomizer comprising an atomizing element. Moreover, in accordance with yet another aspect of the present invention, there is provided an electronic smoking device with an atomizer comprising an atomizing element. The atomizing element is an infrared light source.

The characteristics, features and advantages of this invention and the manner in which they are obtained as described above, will become more apparent and be more clearly understood in connection with the following description of exemplary embodiments, which are explained with reference to the accompanying drawings.

### BRIEF DESCRIPTION OF THE DRAWINGS

In the drawings, same element numbers indicate same elements in each of the views:
Figure 1 is a schematic cross-sectional illustration of an exemplary e-cigarette;
Figure 2 is a schematic cross-sectional view of an enlarged part of an exemplary embodiment of the electronic smoking device;
Figure 3 is a schematic cross-sectional view of an enlarged part of another embodiment of the electronic smoking device; and
Figure 4 is a schematic cross-sectional view of an enlarged part of yet another exemplary embodiment of the electronic smoking device.

### DESCRIPTION OF THE PREFERRED EMBODIMENTS

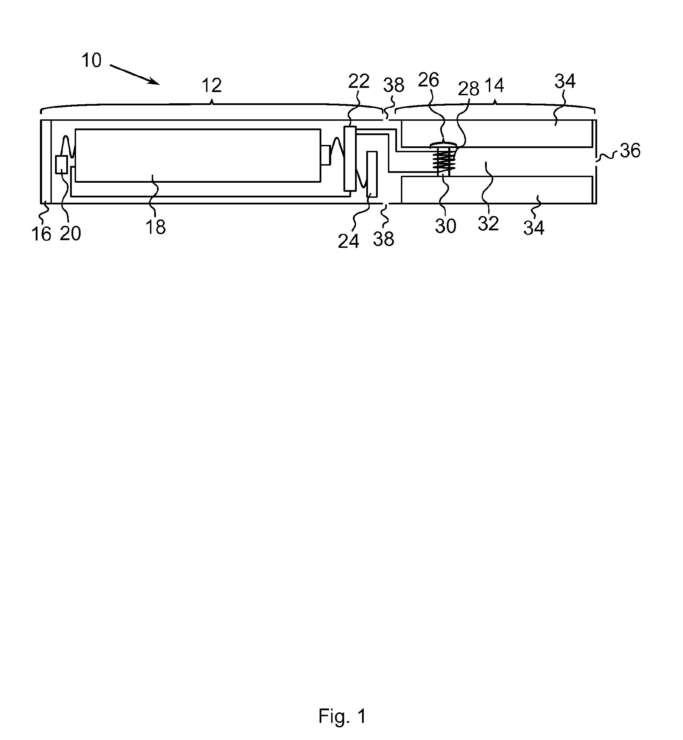

Prior to describing embodiments of the present invention, the basic structure of an electronic smoking device like an e-cigarette will first be described with reference to Figure 1 which is a schematic cross-sectional illustration of an exemplary e-cigarette.

Throughout the following, an electronic smoking device will be exemplarily described with reference to an e-cigarette. As is shown in Figure 1, an e-cigarette 10 typically has a housing comprising a cylindrical hollow tube having an end cap 16. The cylindrical hollow tube may be single piece or a multiple piece tube. In Figure 1, the cylindrical hollow tube is shown as a two piece structure having a battery portion 12 and an atomizer/liquid reservoir portion 14. Together the battery portion 12 and the atomizer/liquid reservoir portion 14 form a cylindrical tube which is approximately the same size and shape as a conventional cigarette, typically about 100 mm with a 7.5 mm diameter, although lengths may range from 70 to 150 or 180 mm, and diameters from 5 to 20 mm.

The battery portion 12 and atomizer/liquid reservoir portion 14 are typically made of steel or hardwearing plastic and act together with the end cap 16 to provide a housing to contain the components of the e-cigarette 10. The battery portion 12 and an atomizer/liquid reservoir portion 14 may be configured to fit together by a friction push fit, a snap fit, or a bayonet attachment, magnetic fit, or screw threads. The end cap 16 is provided at the front end of the battery portion 12. The end cap 16 may be made from translucent plastic or other translucent material to allow an LED 20 positioned near the end cap to emit light through the end cap. The end cap can be made of metal or other materials that do not allow light to pass.

An air inlet may be provided in the end cap, at the edge of the inlet next to the cylindrical hollow tube, anywhere along the length of the cylindrical hollow tube, or at the connection of the battery portion 12 and the atomizer/liquid reservoir portion 14. Figure 1 shows a pair of air inlets 38 provided at the intersection between the battery portion 12 and the atomizer/liquid reservoir portion 14.

A battery 18, a light emitting diode (LED) 20, control electronics 22 and optionally an airflow sensor 24 are provided within the cylindrical hollow tube battery portion 12. The battery 18 is electrically connected to the control electronics 22, which are electrically connected to the LED 20 and the airflow sensor 24. In this example the LED 20 is at the front end of the battery portion 12, adjacent to the end cap 16 and the control electronics 22 and airflow sensor 24 are provided in the central cavity at the other end of the battery 18 adjacent the atomizer/liquid reservoir portion 14.

The airflow sensor 24 acts as a puff detector, detecting a user puffing or sucking on the atomizer/liquid reservoir portion 14 of the e-cigarette 10. The airflow sensor 24 can be any suitable sensor for detecting changes in airflow or air pressure such a microphone switch including a deformable membrane which is caused to move by variations in air pressure. Alternatively the sensor may be a Hall element or an electro-mechanical sensor.

The control electronics 22 are also connected to an atomizer 26. In the example shown, the atomizer 26 includes a heating coil 28 which is wrapped around a wick 30 extending across a central passage 32 of the atomizer/liquid reservoir portion 14. The coil 28 may be positioned anywhere in the atomizer 26 and may be transverse or parallel to the liquid reservoir 34. The wick 30 and heating coil 28 do not completely block the central passage 32. Rather an air gap is provided on either side of the heating coil 28 enabling air to flow past the heating coil 28 and the wick 30. The atomizer may alternatively use other forms of heating elements, such as ceramic heaters, or fiber or mesh material heaters. Nonresistance heating elements such as sonic, piezo and jet spray may also be used in the atomizer in place of the heating coil.

The central passage 32 is surrounded by a cylindrical liquid reservoir 34 with the ends of the wick 30 abutting or extending into the liquid reservoir 34. The wick 30 may be a porous material such as a bundle of fiberglass fibers, with liquid in the liquid reservoir 34 drawn by capillary action from the ends of the wick 30 towards the central portion of the wick 30 encircled by the heating coil 28.

The liquid reservoir 34 may alternatively include wadding soaked in liquid which encircles the central passage 32 with the ends of the wick 30 abutting the wadding. In other embodiments the liquid reservoir 34 may comprise a toroidal cavity arranged to be filled with liquid and with the ends of the wick 30 extending into the toroidal cavity.

An air inhalation port 36 is provided at the back end of the atomizer/liquid reservoir portion 14 remote from the end cap 16. The inhalation port 36 may be formed from the cylindrical hollow tube atomizer/liquid reservoir portion 14 or maybe formed in an end cap.

In use, a user sucks on the e-cigarette 10. This causes air to be drawn into the e-cigarette 10 via one or more air inlets, such as air inlets 38 and to be drawn through the central passage 32 towards the air inhalation port 36. The change in air pressure which arises is detected by the airflow sensor 24 which generates an electrical signal that is passed to the control electronics 22. In response to the signal, the control electronics 22 activate the heating coil 28 which causes liquid present in the wick 30 to be vaporized creating an aerosol (which may comprise gaseous and liquid components) within the central passage 32. As the user continues to suck on the e-cigarette 10, this aerosol is drawn through the central passage 32 and inhaled by the user. At the same time the control electronics 22 also activate the LED 20 causing the LED 20 to light up which is visible via the translucent end cap 16 mimicking the appearance of a glowing ember at the end of a conventional cigarette. As liquid present in the wick 30 is converted into an aerosol more liquid is drawn into the wick 30 from the liquid reservoir 34 by capillary action and thus is available to be converted into an aerosol through subsequent activation of the heating coil 28.

Some e-cigarette are intended to be disposable and the electric power in the battery 18 is intended to be sufficient to vaporize the liquid contained within the liquid reservoir 34 after which the e-cigarette 10 is thrown away. In other embodiments the battery 18 is rechargeable and the liquid reservoir 34 is refillable. In the cases where the liquid reservoir 34 is a toroidal cavity, this may be achieved by refilling the liquid reservoir 34 via a refill port. In other embodiments the atomizer/liquid reservoir portion 14 of the e-cigarette 10 is detachable from the battery portion 12 and a new atomizer/liquid reservoir portion 14 can be fitted with a new liquid reservoir 34 thereby replenishing the supply of liquid. In some cases, replacing the liquid reservoir 34 may involve replacement of the heating coil 28 and the wick 30 along with the replacement of the liquid reservoir 34. A replaceable unit comprising the atomizer 26 and the liquid reservoir 34 is called a cartomizer.

The new liquid reservoir 34 may be in the form of a cartridge having a central passage 32 through which a user inhales aerosol. In other embodiments, aerosol may flow around the exterior of the cartridge 32 to an air inhalation port 36.

Of course, in addition to the above description of the structure and function of a typical e-cigarette 10, variations also exist. For example, the LED 20 may be omitted. The airflow sensor 24 may be placed adjacent the end cap 16 rather than in the middle of the e-cigarette. The airflow sensor 24 may be replaced with a switch which enables a user to activate the e-cigarette manually rather than in response to the detection of a change in air flow or air pressure.

Different types of atomizers may be used. Thus for example, the atomizer may have a heating coil in a cavity in the interior of a porous body soaked in liquid. In this design aerosol is generated by evaporating the liquid within the porous body either by activation of the coil heating the porous body or alternatively by the heated air passing over or through the porous body. Alternatively the atomizer may use a piezoelectric atomizer to create an aerosol either in combination or in the absence of a heater.

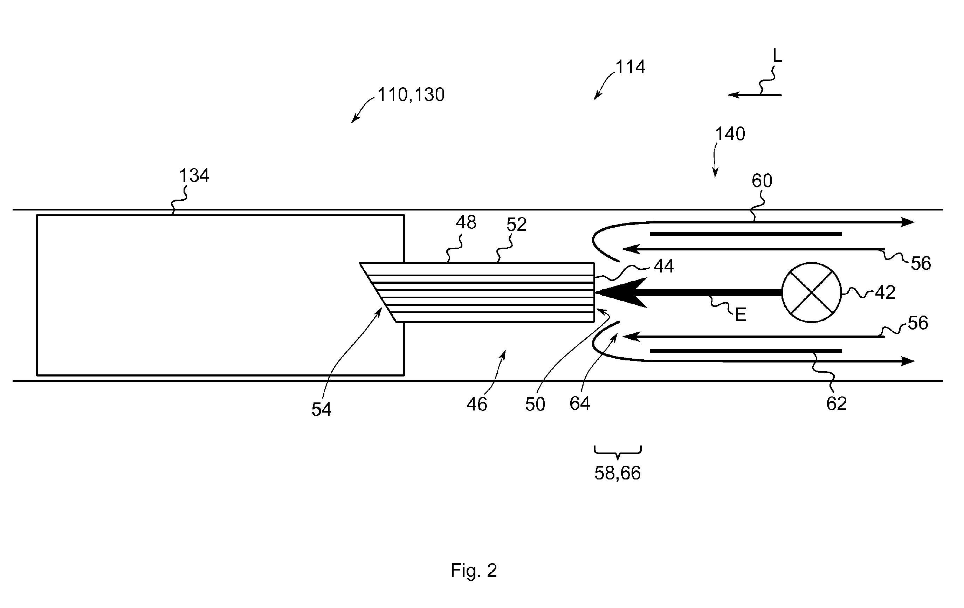

Figure 2 shows a schematic cross-sectional view of an enlarged part of an embodiment of the electronic smoking device 110, e.g. the electronic or e-cigarette, in a partial cross-sectional view. For the sake of brevity, only the differences from the exemplary embodiment of Figure 1 are looked at.

The electronic smoking device 110 is shown with the liquid reservoir 134, such that the embodiment of Figure 2 may also be designated as a sales unit 40, which comprises the electronic smoking device 110, and in addition at least one liquid reservoir 134. The liquid reservoir 134 may be filled or refillable and be placed in the battery portion 12 of the electronic smoking device 10. In case the liquid reservoir 134 is exchangeable, further liquid reservoirs 134 may be provided with the sales unit 40. The liquid reservoir 134 may not be placed in the battery portion 12, but may be provided separate from the electronic smoking device 110.

The electronic smoking device 110 is provided with an atomizer 140 having atomizing element, which is an infrared light source 42 that, during operation of the electronic smoking device 110, emits infrared light in an emitting direction E. Hence, the infrared light source 42 converts electrical energy into electromagnetic energy.

The electronic smoking device 110 comprises a supply element 44 for supplying liquid to be atomized. For example, in the emitting direction E behind the infrared light source 42, the supply element 44 for supplying liquid to be atomized is provided, such that a beam of infrared light emitted by the infrared light source 42 when operated is directed onto the supply element 44. The beam of infrared light heats liquid present in or on the supply element 44, such that the liquid is atomized or vaporized. The supply element 44 has a low absorptivity for light irradiated from the infrared light source 42, such that the supply element 44 itself is not considerably heated by the infrared light emitted from the infrared light source 42. Hence, a typical wavelength of light emitted by the infrared light source 42 is outside of an absorption band of the supply element 44. An absorption band may be a range of wavelengths in the electromagnetic spectrum, which is characteristic of a particular transition from an initial state to a final state in a substance, wherein the change from the initial state to the final state is caused by absorption of radiation, for example emitted by the infrared light source 42. Hence, the supply element 44 itself may not or not completely absorb the infrared radiation emitted by the infrared light source 42, such that the supply element 44 is not unduly heated when irradiated in a dry state. Therefore, overheating of the supply element 44 in the dry state and potentially damaging temperatures of the supply element 44 may be prevented.

The supply element 44 may for example comprise or even consist of a sponge or wick material and supplies the liquid due to capillary action from the liquid reservoir 134 towards the infrared light source 42.

The supply element 44 is part of a liquid supply unit 46 with a housing 48. The supply element 44 is arranged in the housing 48 in order to prevent liquid from dripping off of the supply element 44. The housing 48 comprises a window 50, e.g. an opening, via which light emitted by the infrared light source 42 can readily irradiate the supply element 44. In case evaporated liquid shall exit the supply element 44 via the window 50, the window may be open. Alternatively, the window 50 may be closed by a material that is transparent for light emitted by the infrared light source 42. In case the window 50 is closed, the liquid supply unit 46, for example its housing 48, may comprise at least one opening, via which vaporized liquid can flow.

The housing is depicted formed as a tube 52, which provides the window 50, e.g. at one of its longitudinal ends. The tube 52 has a straight shape. Hence, the supply element 44 can be easily introduced into the tube 52, and the liquid supply unit 46 with the tube 52 can be easily installed into the battery portion 12 in a space saving manner. Due to the tube 52, the liquid reservoir 134 may be arranged at a distance from the infrared light source 42, such that the liquid in the liquid reservoir 134 may not be unduly heated during operation of the infrared light source 42. In case the liquid reservoir 134 is refillable or exchangeable, refilling or exchanging the liquid reservoir 134 may be facilitated due to the distance between the infrared light source 42 and the liquid reservoir 134.

One side of the tube 52 faces the infrared light source 42 and forms the window 50. An opening 54 opposite of the window 50 is arranged or can be arranged in the liquid reservoir 134 in order to receive liquid from the liquid reservoir 134. For example, the opening 54 is formed by a needle point shaped or chamfered end of the tube 52, such that it can easily penetrate the liquid reservoir 134. The needle point shaped or chamfered end of the tube 52 may facilitate an easy atomizer/liquid reservoir portion or exchange of the liquid reservoir 134.

The electronic smoking device 110 comprises an air supply path 56 for supplying air. For example, the air supply path 56 begins at one or all of the air inlets 38 and leads towards the window 50 of the tube 52. At the window 50, air supplied along the air supply path 56 is mixed with atomized or vaporized liquids, such that a mixing volume 58 is formed before the window 50, in particular in the emitting direction E. By mixing the air with the atomized liquid, an aerosol is formed as the inhalant, which is discharged along an inhalant discharge path 60 for discharging atomized material mixed with air supplied via the air supply path 56 and towards the air inhalation port 36 of the electronic smoking device 10. Hence, the air supply path 56 and the inhalant discharge path 60 are interconnected by the mixing volume 58.

The infrared light source 42 is arranged in the air supply path 56, such that atomized materials may not be transported to the infrared light source 42 and cannot contaminate the infrared light source 42. Hence, a technical effect of such an arrangement may be that air supplied along the air supply path 56 flows around the infrared light source 42 and keeps away atomized liquid from the infrared light source 42. Furthermore, the air flowing along the infrared light source 42 may cool the infrared light source 42 during operation, thereby improving aerosol generation and the lifetime of the infrared light source 42 due to prevention of overheating.

The infrared light source 42 is arranged in a sleeve 62. Hence, the infrared light source 42 may be mountable easily and in a space saving manner. Furthermore, the sleeve 62 may prevent radiation emitted by the infrared light source 42 from being directed to other parts of the electronic smoking device 110, which shall not be irradiated. The sleeve 62 is transparent for light emitted by the infrared light source 42 and may open in the emitting direction E, such that a window 64 of the sleeve 62 faces the window 50. Alternatively to the open form of the window 64, the window 64 may be closed by a material that is transparent for the light emitted by the infrared light source 42. In case the window 64 is closed, the air supply path 56 may extend to the mixing volume 58 outside of the sleeve 62. Yet, the infrared light source 42 may be protected from contamination with the vaporized liquid by the closed window 64.

Between the windows 50, 64, a gap 66 remains, into which the air is let via the air supply path 56. Hence, the mixing volume 58 is arranged in the gap 66 between the tube 52 and the sleeve 62. The air supply path 56 extends through or outside of the sleeve 62, for example along or perpendicular to a longitudinal direction L of the electronic smoking device 110. The emitting direction E and the longitudinal direction L may be parallel or antiparallel to each other.

In case the infrared light source 42 and the air supply path 56 are arranged in the sleeve 62, inhalants containing vaporized liquid may not flow through the sleeve 62 and in particular to the infrared light source 42. Hence, the inhalant discharge path 60 is preferably arranged to extend along an outer side of the sleeve 62. Thus, in addition to the flow characteristics along the paths 56, 60, the sleeve 62 may further prevent that vaporized liquid can be deposited on the infrared light source 42.

The inhalant discharge path 60 at least sectionwise or even completely extends in a direction opposite to the air supply path 56. In particular, along the outer side of the sleeve 62, the inhalant discharge path 60 extends opposite to the air supply path 56 in case the user draws a puff from the electronic smoking device 110. Hence, due to the folded arrangement of the paths 56, 60, a dimension of the electronic smoking device 10 along its longitudinal direction L may be reduced. Alternatively, the inhalant discharge path 60 may at least sectionwise or even completely extend in the longitudinal direction L, i.e. parallel to the air supply path 56 and for example along the liquid reservoir 134 towards the air inhalation port.

The typical wavelength of the infrared light source 42 is a wavelength, at which a spectrum of light emitted by the infrared light source 42 has its maximum. The typical wavelength of the infrared light source 42 may be between 1200 nm and 1700 nm, for example between 1400 nm and 1600 nm and for example a wavelength selected from the wavelengths 1400 nm, 1450 nm, 1465 nm, 1475 nm, 1500 nm, 1525 nm, 1550 nm and 1560 nm. A spectral width, for example a half width or a spectral width, at which the emitted power is reduced by 3 decibel, may be between 5 nm and 115 nm, for example 10 nm, 50 nm or 100 nm. The emitted power of the infrared light source 42 during operation may be between 30 mW and 100 mW, for example 60 mW. The infrared light source 42 may be an infrared light-emitting diode (LED) or an infrared semiconductor laser that emits infrared light and in particular near infrared light when operated.

The components shown in Figure 2 may be part of an atomizer/liquid reservoir portion 114 of the electronic smoking device 110. Alternatively, the shown components may be part of a retrofit kit 130 or of an atomizer 140 for the electronic smoking device 110.

The atomizer/liquid reservoir portion 114and the atomizer 140 may be an integral part of the electronic smoking device 110, but, however, may be provided separate from other components of the electronic smoking device 110, in particular separate from the battery 18 and for example also from the battery portion 12 prior to assembling the electronic smoking device 110. The retrofit kit 130 may be provided as a retrofit module, which can be exchanged by another retrofit kit 130. The retrofit kit 130 may also be designated as cartomizer or clearomizer. A cartomizer may differ from a clearomizer by another liquid reservoir 134. The liquid reservoir 134 of the cartomizer may comprise a material that is soaked with the liquid to be atomized or vaporized, such a material for example being wadding or cotton. The liquid reservoir 134 of the clearomizer may be a tank that can be transparent and that does not comprise any material soaked with the liquid to be atomized or vaporized, but rather a free volume for the liquid.

The atomizer/liquid reservoir portion 114 may be attached or attachable to the battery portion 12 of the electronic smoking device 110 or may be an integral part of the electronic smoking device 110. For example, the atomizer/liquid reservoir portion 114 may be insertable into a section of a housing of the electronic smoking device. The atomizer/liquid reservoir portion 114 may be exchangeable against another atomizer/liquid reservoir portion 114, which may be provided with or separate from the electronic smoking device 110 and in particular with or separate from the battery portion 12. The atomizer/liquid reservoir portion 114 may be formed as a cartomizer or as a clearomizer. The atomizer and the liquid reservoir 114 may be combined to form a single piece, e.g. a cartomizer or a clearomizer, or can be provided separate from each other.

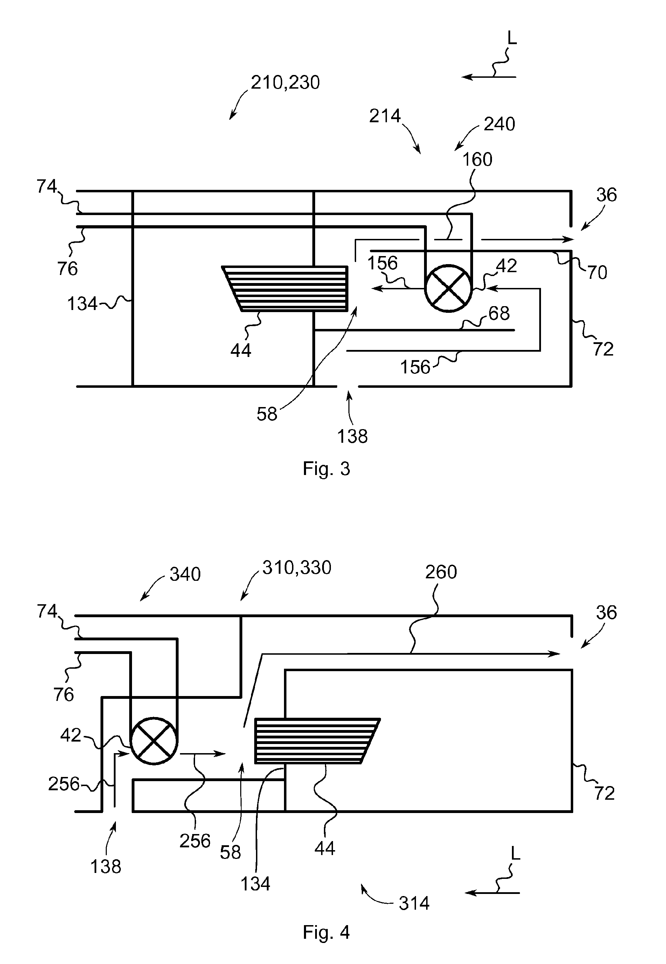

Figure 3 shows a schematic cross-sectional view of an enlarged part of another embodiment of the electronic smoking device 210, e.g. the electronic or e-cigarette, in a partial cross-sectional view. For the sake of brevity, only the differences from the exemplary embodiments of Figures 1 and 2 are looked at.

The exemplary embodiment shown in Figure 3 comprises one air inlet 138. The air supply path 156 of the embodiment of Figure 3 begins at the air inlet 138 and extends to the mixing volume 58. Between the air inlet 138 and the mixing volume 58, the air supply path 156 changes its direction twice, namely from a direction along the longitudinal direction L to a direction perpendicular to the longitudinal direction L and, then, in a direction parallel to the longitudinal direction L. Hence, the air supply path 156 is essentially U-shaped between the air inlet 138 and the mixing volume 58.

In order to guide the supply air along the air supply path 156, a guiding wall 68 is arranged between the infrared light source 42 of the atomizer 240 and the air inlet 138. In addition to forming the air supply path 156, the guide wall 68 blocks light emitted by the infrared light source 42 such that light emitted by the lights source 42 cannot readily exit via the air inlet 138. Hence, undesired loss of light emitted by the lights source 42 to the surroundings is prevented.

From the mixing volume 58, the inhalant discharge path 160 extends towards the air inhalation port 36. Between the mixing volume 58 and the air inhalation port 36, the inhalant discharge path 160 changes its direction from the direction parallel to the longitudinal direction L to a direction perpendicular to the longitudinal direction L and then to a direction along the longitudinal direction L. Hence, the inhalant discharge path 160 of the embodiment of Figure 3 is essentially L-shaped.

In order to block light emitted from the infrared light source 42 from exiting via the air inhalation port 36, another guide wall 70 may be provided. Guide wall 68 may be designated as first wall and guide wall 70 may be designated as second guide wall.

The guide walls 68, 70 comprise free ends, which extend in opposite directions along the longitudinal direction L. For example, the free ends of the first guide wall 68 points against the longitudinal direction L and towards a base 72 of the atomizer/liquid reservoir portion 114, the base being provided with the air inhalation port 36. The second guide wall 70 can comprise a free end that points away from the base 72 in the longitudinal direction L. The infrared light source 42 is arranged between the supply element 44 and the base 72, for example its air inhalation port 36, along the longitudinal direction L. The first guide wall 68 can be based on the liquid reservoir 134. The second guide wall 70 can be based on base 72. Furthermore, the second guide wall 70 can abut on the air inhalation port 36.

Hence, the air supply path 156 and the inhalant discharge path 160 form a meandering gas duct labyrinth.

Furthermore, the embodiment of Figure 3 is shown with energy supply lines, via which operation energy for the infrared light source 42 can be provided from the battery 18. The energy supply lines 74, 76 can contact contact elements for the battery 18. Alternatively, the energy supply lines 74, 76 can be connected to contact elements that are connectable to the battery 18.

The components shown in Figure 3 may be part of an atomizer/liquid reservoir portion 214 of the electronic smoking device 210. Alternatively, the shown components may be part of a retrofit kit 230 or of an atomizer 240 for the electronic smoking device 210.

The atomizer/liquid reservoir portion 214 and the atomizer 240 may be an integral part of the electronic smoking device 210, but, however, may be provided separate from other components of the electronic smoking device 210, in particular separate from the battery 18 and for example also from the battery portion 12 prior to assembling the electronic smoking device 210. The retrofit kit 230 may be provided as a retrofit module, which can be exchanged by another retrofit kit 230. The retrofit kit 230 may also be designated as cartomizer or clearomizer. A cartomizer may differ from a clearomizer by another liquid reservoir 134. The liquid reservoir 134 of the cartomizer may comprise a material that is soaked with the liquid to be atomized or vaporized, such a material for example being wadding or cotton. The liquid reservoir 134 of the clearomizer may be a tank that can be transparent and that does not comprise any material soaked with the liquid to be atomized or vaporized.

The atomizer/liquid reservoir portion 214 may be attached or attachable to the battery portion 12 of the electronic smoking device 210 or may be an integral part of the electronic smoking device 210. For example, the atomizer/liquid reservoir portion 214 may be insertable into a section of a housing of the electronic smoking device. The atomizer/liquid reservoir portion 314 may be exchangeable against another atomizer/liquid reservoir portion 214, which may be provided with or separate from the electronic smoking device 310 and in particular with or separate from the battery portion 12. The atomizer/liquid reservoir portion 214 may be formed as a cartomizer or as a clearomizer. The atomizer and the liquid reservoir 214 may be combined to form a single piece, e.g. a cartomizer or a clearomizer, or can be provided separate from each other.

Figure 4 shows another exemplary embodiment of the invention in a schematic cross-sectional view. For the sake of brevity, only the differences from the exemplary embodiments of Figures 1 to 3 are looked at.

According to the exemplary embodiment of Figure 4, the supply element 44 is arranged between the infrared light source 42 of the atomizer 340 and the base 72 of the atomizer/liquid reservoir portion 314. Thus, in case the liquid reservoir 134 shall be exchanged without exchanging the infrared light source 42, it may suffice to simply exchange the part of the atomizer/liquid reservoir portion 314 that comprises the liquid reservoir 134 with the supply element 44, and the base 72 with the air inhalation port 36.

The air supply path 256 extends from the air inlet 138 towards the infrared light source 42. Between the air inlet 138 and the infrared light source 42, the air supply path 256 essentially extends perpendicular to the longitudinal direction L. In the area of the infrared light source 42, the air supply path 256 changes its direction, such that it extends along and in particular against the longitudinal direction L towards the base 72. In order to avoid that light emitted by the lights source 42 exits via the air inlet 138, the infrared light source 42 may be arranged displaced with respect to the air inlet 138 towards the base 72. Yet, as the infrared light source 42 of all embodiments preferably emits directed light, i.e. it emits light in a preferred direction, the infrared light source 42 may still overlap with a projection of the air inlet 138 perpendicular to the longitudinal direction L.

Thus, the air supply path 256 is essentially L-shaped. A separate guide wall 68 is not necessary, such that the interior of the atomizer/liquid reservoir portion 314 can be produced more easily.

From the mixing volume 58, the inhalant discharge path 260 extends towards the air inhalation port 36. The inhalant discharge path 260 is L-shaped and inhalant is guided around the liquid reservoir 134, such that the second guide wall 70 is provided by the liquid reservoir 134, thereby, again, facilitating producability of the atomizer/liquid reservoir portion 314. The liquid reservoir 134 may abut against the air inhalation port 36 perpendicular to the longitudinal direction L.

The gas duct formed by the air supply path 256 and the inhalant discharge path 260 changes its direction in an alternating manner, such that it first changes its direction in a first, e.g. a clockwise direction, then changes its direction in a second, opposite, e.g. counter-clockwise direction, and furthermore changes its direction again in the first, e.g. clockwise direction. When viewed in the drawing plane, the gas duct has the shape of a profile of two steps.

The components shown in Figure 4 may be part of an atomizer/liquid reservoir portion 314 of the electronic smoking device 310. Alternatively, the shown components may be part of a retrofit kit 330 or of an atomizer 340 for the electronic smoking device 310.

The atomizer/liquid reservoir portion 314 and the atomizer 340 may be an integral part of the electronic smoking device 310, but, however, may be provided separate from other components of the electronic smoking device 310, in particular separate from the battery 18 and for example also from the battery portion 12 prior to assembling the electronic smoking device 310. The retrofit kit 330 may be provided as a retrofit module, which can be exchanged by another retrofit kit 330. The retrofit kit 330 may also be designated as cartomizer or clearomizer. A cartomizer may differ from a clearomizer by another liquid reservoir 134. The liquid reservoir 134 of the cartomizer may comprise a material that is soaked with the liquid to be atomized or vaporized, such a material for example being wadding or cotton. The liquid reservoir 134 of the clearomizer may be a tank that can be transparent and that does not comprise any material soaked with the liquid to be atomized or vaporized, but rather a free volume for the liquid.

The atomizer/liquid reservoir portion 314 may be attached or attachable to the battery portion 12 of the electronic smoking device 310 or may be an integral part of the electronic smoking device 310. For example, the atomizer/liquid reservoir portion 314 may be insertable into a section of a housing of the electronic smoking device. The atomizer/liquid reservoir portion 314 may be exchangeable against another atomizer/liquid reservoir portion 314, which may be provided with or separate from the electronic smoking device 310 and in particular with or separate from the battery portion 12. The atomizer/liquid reservoir portion 314 may be formed as a cartomizer or as a clearomizer. The atomizer and the liquid reservoir 314 may be combined to form a single piece, e.g. a cartomizer or a clearomizer, or can be provided separate from each other.

In the following, the atomizer, the atomizer/liquid reservoir portion, the electronic smoking device and the retrofit kit are further described in an exemplary manner.

In accordance with one aspect of the present invention, there is provided an atomizer for an electronic smoking device, with an atomizing element that is adapted to atomize, e.g. by vaporization, liquid to be inhaled by a user of the electronic smoking device. The atomizing element is an infrared light source. Furthermore, in accordance with another aspect of the present invention, there is provided an electronic smoking device with an atomizer. The atomizer is an atomizer according to the invention. In yet another aspect of the present invention, there is provided an atomizer/liquid reservoir portion for an electric smoking device with a liquid reservoir and an atomizer. The atomizer is an atomizer according to the invention. Moreover, according to another aspect of the present invention, there is provided a retrofit kit for an electronic smoking device. The retrofit kit comprises an atomizer/liquid reservoir portion according to the invention.

Usually, electric heating elements are used to atomize and in particular to vaporize the liquid. However, electric heating elements, for example resistors or coils, which convert electrical in thermal energy, have a low efficiency when vaporizing the liquid, as they heat their complete surroundings. Furthermore, in case no liquid is available, known electric heating elements bear the risk of overheating and thereby damaging the electronic smoking device.

An advantage of the atomizer with the infrared light source as atomizing element may be that the infrared light source mainly heats an area, which is irradiated by infrared light emitted by the infrared light source. For example in comparison with electrical heating elements, other areas than the irradiated area are heated less, such that heating efficiency and, therefore, atomizing or vaporizing efficiency is improved.

The solutions can be combined as desired and further improved by the further following embodiments that are advantageous on their own, in each case and unless stated to the contrary.

The atomizer/liquid reservoir portion may comprise a supply element for supplying liquid to be atomized, in particular towards the infrared light source. The supply element may be adapted to transport liquid due to capillary action, for example from the liquid reservoir towards the infrared light source. A typical wavelength of the infrared light source may be outside of an absorption band of the supply element. Hence, in case the supply element is irradiated by the infrared light source in a dry or merely dry state, the supply element may not be heated or overheated due to the irradiation.

The typical wavelength may be a wavelength with a maximum amplitude in a spectrum of light emitted by the infrared light source. An absorption band may be a range of wavelengths in the electromagnetic spectrum, which is characteristic of a particular transition from an initial state to a final state in a substance, wherein the change from the initial state to the final state is caused by absorption of radiation, in particular emitted by the infrared light source. Hence, the supply element itself may not or not completely absorb the infrared radiation emitted by the infrared light source, such that the supply element is not unduly heated when irradiated in a dry state. Therefore, overheating of the supply element in the dry state and temperatures potentially damaging the supply element may be prevented.

In order to supply the liquid to the infrared light source, the atomizer/liquid reservoir portion may comprise a liquid supply unit with a housing for the supply element, the housing comprising a window via which radiation from the infrared light source irradiates the supply element when the infrared light source is operated. The window may be open, i.e. an opening, or may be closed by a material transparent for light emitted by the infrared light source. Due to the arrangement of the supply element in the housing, liquid dripping off of the supply element may be prevented such that the atomizer/liquid reservoir portion or other components of the electronic smoking device are not contaminated with the liquid and no liquid is wasted. For example, the window may be arranged within a line of sight between the infrared light source and the supply element, the line of sight extending along an emitting direction of the infrared light source.

The housing may be formed as a tube, which provides the window and in which the supply element is arranged. The tube may facilitate the installation of the liquid supply unit. Due to the tube, the liquid reservoir may be arranged at a distance from the infrared light source, such that the liquid in the liquid reservoir may not be unduly heated during operation of the infrared light source. In case the liquid reservoir is refillable or exchangeable, refilling or exchanging the liquid reservoir may be facilitated due to the distance between the infrared light source and the liquid reservoir.

The atomizer/liquid reservoir portion may comprise an air supply path for supplying air, and an inhalant discharge path for discharging atomized material mixed with the air supplied by the air supply path, for example towards a atomizer/liquid reservoir portion of the electronic smoking device, wherein the atomizer/liquid reservoir portion may provide the atomizer/liquid reservoir portion.

The infrared light source may be arranged in the air supply path, such that contamination of the infrared light source with the aerosol generated by atomizing or vaporizing the liquid may be avoided. Hence, a technical effect of such an arrangement may be that air supplied along the air supply path flows around the infrared light source and keeps away atomized liquid from the infrared light source. Furthermore, the air flowing along the infrared light source may cool the infrared light source during operation, thereby improving vaporization and the lifetime of the infrared light source due to prevention of overheating.

For example, the infrared light source is arranged in a sleeve that is at least sectionwise transparent for light emitted by the infrared light source and e.g. opens away from the infrared light source in its emitting direction, wherein the air supply path may extend through the sleeve. The sleeve may comprise two openings that open away from each other, wherein one of the openings is arranged behind the infrared light source in its emitting direction. This opening preferably faces the opening of the tube, such that infrared light emitted by the infrared light source readily can irradiate the liquid supply element. Via the other opening, which opens against the emitting direction, supply air can enter the sleeve when the user sucks on the mouth piece of the electronic smoking device. Arranging the infrared light source in the sleeve may not only prevent the infrared light source from being contaminated with atomized material. Furthermore, the sleeve may prevent radiation emitted by the infrared light source from being directed to other parts of the electronic smoking device, which shall not be irradiated. Finally, the sleeve may provide a space saving way for housing the infrared light source.

The inhalant discharge path may extend along an outer side of the sleeve. Hence, an aerosol transported along the inhalant discharge path may be hindered from reaching the infrared light source, as the infrared light source is screened by the sleeve from the aerosol present in the inhalant.

The inhalant discharge path may at least sectionwise extend in a direction opposite or parallel to the air supply path, in particular in the area of the sleeve and in or against a longitudinal direction of the electronic smoking device. By folding the air supply path and the inhalant discharge path, such that the inhalant discharge path extends opposite to the air supply path, a size and in particular a length of the electronic smoking device can be reduced, in particular in case the air supply path and the inhalant discharge path extend at least sectionwise and for example in the area of the sleeve along the longitudinal direction of the electronic smoking device.

A mixing volume for mixing air with atomized material may be arranged before the opening of the supply element. In particular in the emitting direction of the infrared light source, the mixing volume is arranged before the opening of the liquid supply unit and may abut the opening of the tube and the opening of the sleeve facing the tube. For example, the mixing volume may be formed by a gap between the tube and the sleeve. In order to mix the atomized liquid with the air in a space saving way, the mixing volume may interconnect the air supply path and the inhalant discharge path. Hence, no further paths may be necessary in order to supply the air to the atomized liquid and to discharge the inhalants, i.e. the aerosol formed by the atomized liquid and the air.

In case the atomizer/liquid reservoir portion or the electronic smoking device comprises liquid to be vaporized and inhaled, or is provided with liquid, e.g. within at least one liquid reservoir, e.g. as the sales unit, a typical wavelength of the infrared light source is preferably within an absorption band of the liquid. Hence, it may be possible to atomize and in particular to vaporize the liquid more efficiently compared with electrical heating elements that may transfer heat due to direct contact with the liquid.

The infrared light source may be an infrared light source emitting light in the infrared or the near infrared spectrum. The typical wavelength of the infrared light source may be between 1200 nm and 1700 nm, for example between 1400 nm and 1600 nm and for example a wavelength selected from the wavelengths 1400 nm, 1450 nm, 1465 nm, 1475 nm, 1500 nm, 1525 nm, 1550 nm and 1560 nm. A spectral width, for example a half width or a spectral width, at which the emitted power is reduced by 3 decibel, may be between 5 nm and 115 nm, for example 10 nm, 50 nm or 100 nm. The emitted power of the infrared light source may be between 30 mW and 100 mW, for example 60 mW. The infrared light source is preferably an infrared light-emitting diode (LED) or an infrared semiconductor laser.

The components described part of an atomizer/liquid reservoir portion of the electronic smoking device.

The atomizer/liquid reservoir portion may be an integral part of the electronic smoking device, but, however, may be provided separate from other components of the electronic smoking device, in particular separate from the battery and for example also from the battery portion prior to assembling the electronic smoking device. The retrofit kit may be provided as a retrofit module, which can be exchanged by another retrofit kit. The retrofit kit may also be designated as cartomizer or clearomizer. A cartomizer may differ from a clearomizer by another liquid reservoir. The liquid reservoir of the cartomizer may comprise a material that is soaked with the liquid to be atomized or vaporized, such a material for example being wadding or cotton. The liquid reservoir of the clearomizer may be a tank that can be transparent and that is formed with a free volume for the liquid.

The atomizer/liquid reservoir portion may comprise one air inlet. The air supply path can begin at the air inlet and extend to the mixing volume. Between the air inlet and the mixing volume, the air supply path may change its direction from a direction along the longitudinal direction to a direction perpendicular to the longitudinal direction and, then, in a direction parallel to the longitudinal direction. Hence, the air supply path may be essentially U-shaped between the air inlet and the mixing volume.

In order to guide the supply air along the air supply path, a guiding wall can be arranged between the infrared light source and the air inlet. In addition to forming the air supply path, the guide wall may block light emitted by the infrared light source such that light emitted by the lights source cannot exit via the air inlet. Hence, undesired loss of light emitted by the lights source to the surroundings may be prevented.

From the mixing volume, the inhalant discharge path extends towards the air inhalation port. Between the mixing volume and the air inhalation port, the inhalant discharge path can change its direction from a direction perpendicular to the longitudinal direction to a direction along the longitudinal direction. Hence, the inhalant discharge path may be essentially L-shaped.

In order to block light emitted from the infrared light source from exiting via the air inhalation port, another guide wall may be provided. This guide wall may be designated as first wall, and the previously mentioned guide wall may be designated as second guide wall.

The guide walls comprise free ends, which can extend in opposite directions along the longitudinal direction. For example, the free end of the first guide wall points against the longitudinal direction and towards a base of the atomizer/liquid reservoir portion, the base being provided with the air inhalation port. The second guide wall can comprise a free end that points away from the base in the longitudinal direction. The first guide wall can be based on the liquid reservoir. The second guide wall can be based on the base of the atomizer/liquid reservoir portion. Furthermore, the second guide wall can abut on the air inhalation port. The infrared light source can be arranged between the supply element and the base, for example its air inhalation port along the longitudinal direction.

Hence, the air supply path and the inhalant discharge path may form a meandering gas duct labyrinth.

Furthermore, energy supply lines can be provided, via which operation energy for the infrared light source can be provided from the battery. The energy supply lines can contact contact elements for the battery. Alternatively, the energy supply lines can be connected to contact elements that are connectable to the battery.

The supply element can be arranged between the infrared light source and the base of the atomizer/liquid reservoir portion. Thus, in case the liquid reservoir shall be exchanged without exchanging the infrared light source, it may suffice to simply exchange the part of the atomizer/liquid reservoir portion that comprises the liquid reservoir with the supply element, and the base with the air inhalation port.

The air supply path may extend from the air inlet towards the infrared light source. Between the air inlet and the infrared light source, the air supply path can essentially extend perpendicular towards the longitudinal direction. In the area of the infrared light source, the air supply path may change its direction, such that it extends along and in particular against the longitudinal direction towards the base. In order to avoid that light emitted by the lights source exits via the air inlet, the infrared light source may be arranged displaced with respect to the air inlet towards the base. Yet, as the infrared light source of all embodiments preferably emits directed light, i.e. it emits light in a preferred direction, the infrared light source may still overlap a projection of the air inlet perpendicular to the longitudinal direction.

Thus, the air supply path can be essentially L-shaped. A separate guide wall is not necessary, such that the interior of the atomizer/liquid reservoir portion can be produced more easily.

From the mixing volume, the inhalant discharge path extends towards the air inhalation port. The inhalant discharge path may be L-shaped and inhalant can be guided around the liquid reservoir, such that the second guide walls not necessary, thereby, again, facilitating producability of the atomizer/liquid reservoir portion. The liquid reservoir may abut against the air inhalation port perpendicular to the longitudinal direction.

The gas duct formed by the air supply path and the inhalant discharge path, can change its direction in an alternating manner, such that it first changes its direction in a first, e.g. a clockwise direction, then changes its direction in a second, opposite, e.g. counter-clockwise direction, and furthermore changes its direction against in the first, e.g. clockwise direction. When viewed perpendicular to the longitudinal direction, the gas duct may have the shape of a profile of two steps.

While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims.

The atomizer/liquid reservoir portion may be attached or attachable to the battery portion of the electronic smoking device or may be an integral part of the electronic smoking device. For example, the atomizer/liquid reservoir portion may be insertable into a section of the housing of the electronic smoking device. The atomizer/liquid reservoir portion may be exchangeable against another atomizer/liquid reservoir portion, which may be provided with or separate from the electronic smoking device and in particular with or separate from the battery portion. The atomizer/liquid reservoir portion may be formed as a cartomizer or as a clearomizer. The atomizer and the liquid reservoir may be combined to form a single piece, e.g. a cartomizer or a clearomizer, or can be provided separate from each other.

### LIST OF REFERENCE SIGNS

- 10, 110, 210, 310: e-cigarette / electronic smoking device
- 12: battery portion
- 14, 114, 214, 314: atomizer/liquid reservoir portion
- 16: end cap
- 18: battery
- 20: light emitting diode (LED)
- 22: control electronics
- 24: airflow sensor
- 26: atomizer
- 28: heating coil
- 30: wick
- 32: central passage
- 34, 134: liquid reservoir
- 36: air inhalation port
- 38, 138: air inlets
- 40: sales unit
- 42: infrared light source
- 44: supply element
- 46: liquid supply unit
- 48: housing
- 50: window of housing
- 52: tube
- 54: opening
- 56, 156, 256: air supply path
- 58: mixing volume
- 60, 160, 260: inhalant discharge path
- 62: sleeve
- 64: window of sleeve
- 66: gap
- 68, 70: guide wall
- 72: base of atomizer/liquid reservoir portion
- 74, 76: energy supply lines
- 130, 230, 330: retrofit kit
- 140, 240, 340: atomizer

- E: emitting direction
- L: longitudinal direction

## Claims

1. Atomizer (140) for an electronic smoking device (110), with an atomizing element that is adapted to atomize liquid to be inhaled by a user of the electronic smoking device (10), wherein the atomizing element is an infrared light source (42).

2. Atomizer/liquid reservoir portion (114) for an electronic smoking device (110), with a liquid reservoir (134) and an atomizer (140), wherein the atomizer (140) is an atomizer (140) according to claim 1.

3. Atomizer/liquid reservoir portion (114) according to claim 2, wherein the atomizer/liquid reservoir portion (114) comprises a supply element (44) for supplying liquid to be atomized, wherein a typical wavelength of the infrared light source (42) is outside of an absorption band of the supply element (44).

4. Atomizer/liquid reservoir portion (114) according to claim 3, wherein the atomizer/liquid reservoir portion (114) comprises a liquid supply unit (46) with a housing (48) for the supply element (44), the housing (48) comprising a window (50), via which the supply element (44) is irradiatable with light from the infrared light source (42).

5. Atomizer/liquid reservoir portion (114) according to claim 4, wherein the housing (48) is a tube (52), which provides the window (50), and in which the supply element (44) is arranged.

6. Atomizer/liquid reservoir portion (114) according to any of claims 2 to 5, wherein the atomizer/liquid reservoir portion (114) comprises an air supply path (56) for supplying air, and an inhalant discharge path (60) for discharging atomized material mixed with air supplied via the air supply path (56), wherein the infrared light source (42) is arranged in the air supply path (56).

7. Atomizer/liquid reservoir portion (114) according to claim 6, wherein the infrared light source (42) is arranged in a sleeve (62) that opens away from the infrared light source (42) in its emitting direction (E), wherein the air supply path (56) extends through the sleeve (62).

8. Atomizer/liquid reservoir portion (114) according to claim 7, wherein the inhalant discharge path (60) extends along an outer side of the sleeve (62).

9. Atomizer/liquid reservoir portion (114) according to any of claims 6 to 8 **characterized in that** the inhalant discharge path (60) at least sectionwise extends in a direction opposite to the air supply path (56).

10. Atomizer/liquid reservoir portion (214) according to claim 6, wherein the air supply path (156) and the inhalant discharge path (160) form a meandering gas duct labyrinth.

11. Atomizer/liquid reservoir portion (314) according to claim 6, wherein the air supply path (256) and the inhalant discharge path (260) form a gas duct that changes its direction in an alternating manner.

12. Atomizer/liquid reservoir portion (114) according to any of claims 6 to 11, **characterized in that** a mixing volume (58) is arranged before the opening (50) of the liquid supply unit (46), the mixing volume (58) interconnecting the air supply path (56) and the inhalant discharge path (60).

13. Electronic smoking device (110) with an atomizer (140), wherein the atomizer (140) is an atomizer (140) according to claim 1.

14. Electronic smoking device (110) according to claim 11, with an atomizer/liquid reservoir portion comprising the atomizer (140), wherein the atomizer/liquid reservoir portion is an atomizer/liquid reservoir portion according to any of claims 2 to 12.
